# EUROPÄISCHE PATENTSCHRIFT

(11) **EP 0 706 800 B1**
(45) Veröffentlichungstag und Bekanntmachung des Hinweises auf die Patenterteilung: **30.08.2000**
(21) Anmeldenummer: 95115435.0
(22) Anmeldetag: 29.09.1995
(51) Int. Cl.: A61L 2/06, A61L 2/20

(54) **Gerät zur Sterilisation bzw. Desinfektion**
Apparatus for sterilisation or disinfection
Appareil pour la stérilisation ou la désinfection

(30) Priorität: 14.10.1994 DE 9416571 U
(43) Veröffentlichungstag der Anmeldung: 17.04.1996
(73) Patentinhaber: MMM MÜNCHENER MEDIZIN MECHANIK GMBH, D-81369 München (DE)
(72) Erfinder: Hofmann, Ludwig, Dipl.-Ing., D-81377 München (DE)
(74) Vertreter: Grünecker, Kinkeldey, Stockmair & Schwanhäusser Anwaltssozietät

(56) Entgegenhaltungen:
- DE-A- 2 313 484
- DE-U- 9 416 571
- GB-A- 1 437 828
- US-A- 4 301 113

## Beschreibung

Die Erfindung betrifft ein Gerät zur Sterilisation bzw. Desinfektion gemäß dem Oberbegriff des Patentanspruchs 1.

Aus DE-A-23 13 484 ist ein derartiges Gerät bekannt. Bei diesem herkömmlichen Gerät ist innerhalb einer Sterilisierkammer ein Axialgebläse vorgesehen, das im Bodenbereich der Sterilisierkammer angeordnet ist und von einer Lochblechanordnung abgedeckt ist. Bei Inbetriebnahme des Axialgebläses wird ein in der Sterilisierkammer befindliches gasförmiges Medium, beispielsweise über einen Randbereich der Lochblechanordnung, angesaugt und über einen Zentralbereich der Lochblechanordnung in den eigentlichen Behandlungsabschnitt zurückgeführt.

Aus US-A-4,301,113 ist ebenfalls ein Gerät zur Durchführung einer Sterilisationsbehandlung bekannt, bei welchem über eine Rohrleitungsanordnung ein in einer Sterilisierkammer befindliches Gas angesaugt und anschließend über Rohrleitungsabschnitte in die Sterilisierkammer zurückgeführt werden kann. Zur Förderung des angesaugten Gases durch die genannte Rohrleitungsanordnung ist ein Gebläse vorgesehen, das außerhalb des Sterilisationsgerätes in der Rohrleitungsanordnung vorgesehen ist und die Wiedereinleitung des Gases durch gegenüberliegende Seitenwände in der Nähe der Verschlußeinrichtung erfolgt.

Der Erfindung liegt die Aufgabe zugrunde, ein Gerät zur Sterilisation bzw. Desinfektion der eingangs genannten Art zu schaffen, bei welchem auf gerätetechnisch günstige Weise eine verbesserte Umwälzung eines in der Sterilisierkammer befindlichen Mediums erreicht werden kann.

Diese Aufgabe wird erfindungsgemäß durch ein Gerät mit den im Patentanspruch 1 angegebenen Merkmalen gelöst.

Dadurch wird es auf vorteilhafte Weise möglich, das im Inneren der Kammer befindliche Fluid derart aktiv umzuwälzen, daß insbesondere bei einem Dampfsterilisationsverfahren, bei welchem grundsätzlich keine Fluidumwälzung im Inneren der Kammer erforderlich ist jene zwischen dem Sattdampf und dem zu behandelnden Gut stattfinden Wärmeausgleichsvorgange unterstützt werden. In besonders vorteilhafter Weise wird es durch die erfindungsgemäße technische Lehre möglich auch herkömmliche Dampfsterilisationsons- bzw. Dampfdesinfektionsgeräte hinsichtlich ihrer Leistungsfähigkeit nachträglich zu verbessern. In vorteilhafter Weise kann dabei bei einem Nachrüsten herkömmlicher Geräte auf einen im allgemeinen arbeits- und zeitaufwendigen Ausbau der Geräte aus einer Schrankwand oder einer Stützstruktur verzichtet werden. In ebenfalls vorteilhafter Weise wird es durch die erfindungsgemäße technische Lehre auch möglich, jene, eine besondere Sorgfalt erfordernde Montageschritte bereits werksseitig vorzunehmen und eine vollständige, auf Funktionssicherheit geprüfte Baugruppe in der Art eines Austauschteiles bereitzuhalten.

Bei dem erfindungsgemäßen Gerät kann auf vorteilhafte Weise in dessen Inneren eine im wesentlichen homogene Atmosphäre erzeugt werden. Diese erfindungsgemäß möglich werdende Homogenisierung erweist sich nicht nur hinsichtlich einer Steigerung der Behandlungseffektivität als besonders vorteilhaft, sondern erlaubt zudem eine besonders zuverlässige Beurteilung des zu erwartenden Behandlungserfolges, da es auf überraschend einfache Weise möglich wird, eine für die Beurteilung des Behandlungserfolges besonders repräsentative Fluidprobe, insbesondere Dampfprobe, zu gewinnen.

Eine bevorzugte Ausführungsform des erfindungsgemäßen Gerätes ist dadurch gegeben, daß die Gebläseeinrichtung ein Querstromgebläse ist. Eine derartige Gebläseeinrichtung ist preiwert herstellbar und relativ platzsparend in die Verschlußeinrichtung integrierbar. Alternativ zu jenem Querstromgebläse ist es auch möglich, die Gebläseeinrichtung als Radial- oder Axialgebläse auszubilden.

Zur Steigerung der Leistungsfähigkeit der Gebläseeinrichtung erweist es sich als vorteilhaft, daß diese mehrere Laufräder umfaßt.

Gemäß einer bevorzugten Ausführungsform der Erfindung ist die Förderrichtung der jeweiligen Gebläse umkehrbar. Dadurch wird es möglich, eine dem jeweiligen Befüllungsgrad der Kammer angepaßte Umwälz- bzw. Zwangskonvektionsform im Inneren der Kammer auszubilden.

In vorteilhafter Weise ist die Verschlußvorrichtung als Türe ausgebildet. Diese Türe ist dabei gemäß einer bevorzugten Ausführungsform der Erfindung als horizontal oder vertikal bewegbare Schiebetüre ausgebildet. Es ist jedoch in vorteilhafter Weise, auch möglich, die Türe als schwenk-bewegbare Türe auszubilden.

Gemäß einer bevorzugten Ausführungsform der Erfindung ist die Gebläseeinrichtung zumindest teilweise versenkt in der Türe angeordnet. Dadurch wird auf vorteilhafte Weise eine Verminderung des im Inneren der Kammer zur Verfügung stehenden Raumes vermieden.

Gemäß einer bevorzugten Ausführungsform ist die Gebläseeinrichtung, insbesondere das Querstromgebläse in einem vorzugsweise oberen Randbereich der Türe angeordnet. Dadurch läßt sich eine fertigungstechnisch besonders günstig herstellbare und betriebssichere Ausführungsform des erfindungsgemäßen Gerätes realisieren.

Eine bevorzugte Ausführungsform des erfindungsgemäßen Gerätes ist dadurch gegeben, daß die Türe mit Fluidleitungseinrichtungen versehen ist, zur Zuführung und/oder Ableitung von Fluid zu bzw. von der Gebläseeinrichtung. Diese Fluidleitungseinrichtungen sind in vorteilhafter Weise mittels eines Tiefziehverfahrens auf der Innenseite der Kammer ausgebildet. In vorteilhafter Weise sind diese Fluidleitungseinrichtungen hinsichtlich ihrer Fluidleitungseigenschaften variierbar, insbesondere absperrbar. Dadurch wird es unabhängig von einer Änderung der Betriebsparameter der Gebläseeinrichtung möglich, Einfluß auf jene im Inneren der Kammer zu erzeugende Umwälz- bzw. Konvektionsform zu nehmen. In vorteilhafter Weise stehen dabei die im Inneren der Türe vorgesehenen Fluidleitungseinrichtungen mit weiteren Fluidleitungseinrichtungen, die im Inneren der Kammer angeordnet sind, in Verbindung. Dadurch wird es auf vorteilhafte Weise möglich, ein im Inneren der Kammer befindliches Fluid aus einem von der Gebläseeinrichtung entfernten Bereich der Kammer abzusaugen und eine besonders effektive Umwälzung und Homogenisierung des im Inneren der Kammer befindlichen Fluides zu ermöglichen. Dadurch wird es auch bei hohen Füllungsgraden möglich, eine hohe Behandlungssicherheit zu gewährleisten.

Weitere Merkmale und Einzelheiten der Erfindung ergeben sich aus der nachfolgenden Beschreibung eines Ausführungsbeispieles in Verbindung mit der Zeichnung. Es zeigen:

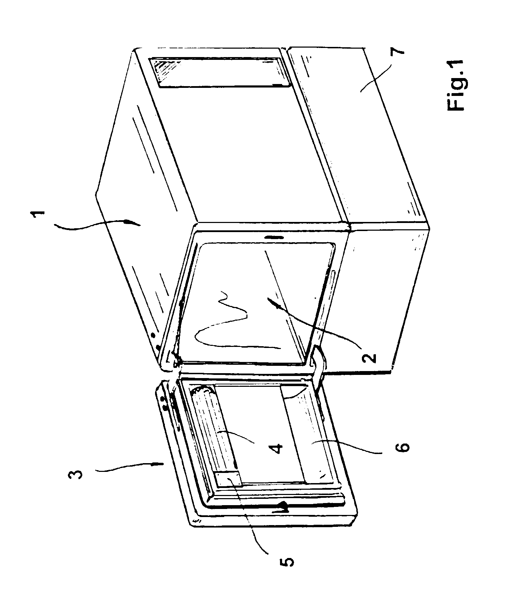

- **Fig. 1**: eine perspektivische Ansicht eines Ausführungsbeispieles des erfindungsgemäßen Gerätes mit einer, in eine Offenstellung geschwenkten Türe.

Gemäß der in Fig. 1 dargestellten Ausführungsform des erfindungsgemäßen Gerätes umfaßt dieses ein Gehäuse 1 in dessen Inneren eine Kammer 2, zur Aufnahme von, im Rahmen eines Sterilisationsverfahrens zu behandelndem Gut, ausgebildet ist. Die Kammer 2 ist mittels einer schwenkbewegbar an dem Gehäuse 1 angelenkten Türe 3 verschließbar. Die Türe 3 ist mit einer Gebläseeinrichtung 4 versehen. Die Gebläseeinrichtung ist in diesem Fall als Querstromgebläse ausgebildet. Der Antrieb des Querstromgebläses erfolgt vermittels einer Antriebseinrichtung 5, welche zugleich eine Achse eines Laufrades des Querstromgebläses lagert. Eine der Antriebseinrichtung 5 gegenüberliegende Seite des Laufrades des Querstromgebläses ist mittels eines Wälzlagers im Gehäuse der Türe 3 gelagert. Über einen Kanal 6 kann Fluid aus der Kammer 2 angesaugt weren. Der Kanal 6 ist wie das Laufrad des Querstromgebläses in die Türe 3 integriert. Das Gehäuse 1 des Gerätes ist auf einen unteren Gehäuseblock 7 aufgesetzt, in welchem eine Sattdampferzeugungseinrichtung sowie eine Vakuumpumpe untergebracht sind.

Das vorstehend beschriebene Gerät kann beispielsweise in der nachfolgend näher erläuterten Weise verwendet werden:

In eine zur Aufnahme von Sterilisiergut vorgesehene Sterilisationskammer wird Sterilisiergut, das im Rahmen eines Dampfsterilisationsverfahrens zu behandeln ist, eingebracht. Nach Einbringen des Sterilisiergutes wird die Sterilisationskammer verschlossen. Über eine Vakuumpumpe wird der Druck im Inneren der Kammer auf einen bestimmten Druckwert abgesenkt. Anschließend wird in die unter vermindertem Druck stehende Sterilisationskammer Sattdampf eingleitet. Während der Einleitung von Sattdampf in die Kammer bzw. im Anschluß an das Einleiten des Dampfes in die Kammer wird die Gebläseeinrichtung in Betrieb genommen. Die Gebläseeinrichtung saugt aus einem unteren Bereich der Kammer jenes bereits in der Kammer befindliche Fluid an und fördert dieses zu dem oberen Bereich der Kammer. Dadurch wird das im Inneren der Kammer befindliche Fluid zwangsweise umgewälzt. Im Rahmen dieser Zwangsumwälzung kann in vorteilhafter Weise eine Fluidanalyse vorgenommen werden. Zur Durchführung dieser Fluid- bzw. Dampfanalyse wird der Kammer eine Dampfprobe entnommen und diese, insbesondere im Hinblick auf deren Gehalt an nicht-kondensierbaren Gasen zur Beurteilung der Verfahrensqualität, analysiert. Nach Abschluß dieser Dampfanalyse wird das im Inneren der Kammer befindliche Sterilisiergut getrocknet. Auch bei diesem Trocknungsvorgang wird in vorteilhafter Weise die Gebläseeinrichtung in Betrieb gesetzt. Nach Abschluß der Sterilisationsbehandlung wird die Türe geöffnet und das in der Kammer befindliche nunmehr sterilisierte Gut der Kammer entnommen.

Die Erfindung ist nicht auf das vorangehend beschriebene Ausführungsbeispiel beschränkt. Beispielsweise ist es auch möglich, anstelle des Querstromgebläses ein Axialgebläse zu verwenden.

## Patentansprüche

1. Gerät zur Sterilisation bzw. Desinfektion mit einer Kammer zur Aufnahme von im Rahmen eines Sterilisations- bzw. Desinfektionsverfahrens zu behandelndem Gut und einer relativ zu der Kammer (2) bewegbaren, sowohl in eine Offenstellung als auch in eine Schließstellung bringbaren Verschlußeinrichtung und einer Gebläseeinrichtung (4) zur Umwälzung von im Inneren der Kammer (2) befindlichem Fluid, **dadurch gekennzeichnet,** daß die Verschlußeinrichtung mit der Gebläseeinrichtung (4) versehen ist.

2. Gerät zur Sterilisation bzw. Desinfektion nach Anspruch 1, **dadurch gekennzeichnet,** daß die Gebläseeinrichtung (4) ein Querstromgebläse ist.

3. Gerät zur Sterilisation bzw. Desinfektion nach Anspruch 1, **dadurch gekennzeichnet,** daß die Gebläseeinrichtung (4) ein Radial- oder Axialgebläse ist.

4. Gerät zur Sterilisation bzw. Desinfektion nach mindestens einem der Ansprüche 1 bis 3, **dadurch gekennzeichnet,** daß die Gebläseeinrichtung (4) mehrere Laufräder umfaßt.

5. Gerät zur Sterilisation bzw. Desinfektion nach mindestens einem der Ansprüche 1 bis 4, **dadurch gekennzeichnet,** daß die Förderrichtung der jeweiligen Gebläseeinrichtungen bzw. Gebläse umkehrbar ist.

6. Gerät zur Sterilisation bzw. Desinfektion nach mindestens einem der Ansprüche 1 bis 5, **dadurch gekennzeichnet,** daß Verschlußvorrichtung als Türe (3) ausgebildet ist.

7. Gerät zur Sterilisation bzw. Desinfektion nach mindestens einem der Ansprüche 1 bis 6, **dadurch gekennzeichnet,** daß die Türe (3) schwenkbewegbar gehaltert ist.

8. Gerät zur Sterilisation bzw. Desinfektion nach mindestens einem der Ansprüche 1 bis 7, **dadurch gekennzeichnet,** daß die Gebläseeinrichtung (4) zumindest teilweise in der Türe (3) versenkt angeordnet ist.

9. Gerät zur Sterilisation bzw. Desinfektion nach mindestens einem der Ansprüche 1 bis 8, **dadurch gekennzeichnet,** daß die Gebläseeinrichtung (4) insbesondere das Querstromgebläse in einem oberen Randbereich der Türe (3) angeordnet ist.

10. Gerät zur Sterilisation bzw. Desinfektion nach mindestens einem der Ansprüche 1 bis 9, **dadurch gekennzeichnet,** daß die Türe (3) mit Fluidleitungseinrichtungen (6) versehen ist zur Zuführung und/oder Ableitung von Fluid zu bzw. von der Gebläseeinrichtung (4).

11. Gerät zur Sterilisation bzw. Desinfektion nach mindestens einem der Ansprüche 1 bis 10, **dadurch gekennzeichnet,** daß die Fluidleitungseinrichtungen (6) hinsichtlich ihrer Fluidleitungseigenschaften variierbar, insbesondere absperrbar, sind.

12. Gerät zur Sterilisation bzw. Desinfektion nach mindestens einem der Ansprüche 1 bis 11, **dadurch gekennzeichnet,** daß die in der Türe (3) vorgesehenen Fluidleitungseinrichtungen (6) mit weiteren im Inneren der Kammer angeordneten Fluidleitungseinrichtungen, beispielsweise einem im unteren Eckbereich der Kammer verlaufenden und zu einem der Verschlußvorrichtung abgewandten Ende der Kammer führenden Kanal in Fluidverbindung stehen.

## Claims

1. Apparatus for sterilisation or disinfection with a chamber for receiving material which is to be treated within the framework of a sterilisation or disinfection method, and a closing device, which is displaceable relative to the chamber (2) and can be brought into both an open position and into a closed position, and a fan device (4) for circulating fluid located in the interior of the chamber (2), characterised in that the closure device is provided with the fan device (4).

2. Apparatus for sterilisation or disinfection according to claim 1, characterised in that the fan device (4) is a cross flow fan.

3. Apparatus for sterilisation or disinfection according to claim 1, characterised in that the fan device (4) is a radial or axial fan.

4. Apparatus for sterilisation or disinfection according to at least one of claims 1 to 3, characterised in that the fan device (4) comprises a plurality of impellers.

5. Apparatus for sterilisation or disinfection according to at least one of claims 1 to 4, characterised in that the conveying direction of the respective fan devices or fan is reversible.

6. Apparatus for sterilisation or disinfection according to at least one of claims 1 to 5, characterised in that the closure device is constructed as a door (3).

7. Apparatus for sterilisation or disinfection according to at least one of claims 1 to 6, characterised in that the door (3) is mounted so as to be pivotable.

8. Apparatus for sterilisation or disinfection according to at least one of claims 1 to 7, characterised in that the fan device (4) is arranged at least partially recessed in the door (3).

9. Apparatus for sterilisation or disinfection-according to at least one of claims 1 to 8, characterised in that the fan device (4), more particularly the cross flow fan, is arranged in an upper peripheral region of the door (3).

10. Apparatus for sterilisation or disinfection according to at least one of claims 1 to 9, characterised in that the door (3) is provided with fluid conducting devices (6) for supplying and/or extracting fluid to or from the fan device (4).

11. Apparatus for sterilisation or disinfection according to at least one of claims 1 to 10, characterised in that the fluid conducting devices (6) are variable, more particularly closable, in respect of their fluid conducting properties.

12. Apparatus for sterilisation or disinfection according to at least one of claims 1 to 11, characterised in that the fluid conducting devices (6) provided in the door (3) communicate for the flow of fluid with further fluid conducting devices arranged within the chamber, for example a duct extending in the lower corner region of the chamber and leading to an end of the chamber remote from the closure device.

## Revendications

1. Appareil de stérilisation ou de désinfection comportant une chambre destinée à recevoir un matériau devant être traité dans le cadre d'un procédé de stérilisation ou de désinfection et un dispositif de fermeture pouvant être déplacé par rapport à la chambre (2) et être amené aussi bien dans une position d'ouverture que dans une position de fermeture, et un dispositif à ventilateur (4) destiné à faire circuler un fluide se trouvant à l'intérieur de la chambre (2), **caractérisé** en ce que le dispositif de fermeture est équipé du dispositif à ventilateur (4).

2. Appareil de stérilisation ou de désinfection selon la revendication 1, **caractérisé** en ce que le dispositif à ventilateur (4) est un ventilateur à courant transversal.

3. Appareil de stérilisation ou de désinfection selon la revendication 1, **caractérisé** en ce que le dispositif à ventilateur (4) est un ventilateur radial ou axial.

4. Appareil de stérilisation ou de désinfection selon au moins une des revendications 1 à 3, **caractérisé** en ce que le dispositif à ventilateur (4) comprend plusieurs roues.

5. Appareil de stérilisation ou de désinfection selon au moins une des revendications 1 à 4, **caractérisé** en ce que l'on peut inverser le sens de refoulement des dispositifs à ventilateur ou des ventilateurs.

6. Appareil de stérilisation ou de désinfection selon au moins une des revendications 1 à 5, **caractérisé** en ce que le dispositif de fermeture est réalisé sous forme de porte (3).

7. Appareil de stérilisation ou de désinfection selon au moins une des revendications 1 à 6, **caractérisé** en ce que la porte (3) est fixée avec possibilité de pivotement.

8. Appareil de stérilisation ou de désinfection selon au moins une des revendications 1 à 7, **caractérisé** en ce que le dispositif à ventilateur (4) est au moins en partie noyé dans la porte (3).

9. Appareil de stérilisation ou de désinfection selon au moins une des revendications 1 à 8, **caractérisé** en ce que le dispositif à ventilateur (4), notamment le ventilateur à courant transversal, est disposé dans la région d'un bord supérieur de la porte (3).

10. Appareil de stérilisation ou de désinfection selon au moins une des revendications 1 à 9, **caractérisé** en ce que la porte (3) est équipée de dispositifs de canalisation de fluide (6) pour amener du fluide vers le dispositif à ventilateur (4) et/ou pour évacuer du fluide du dispositif à ventilateur (4).

11. Appareil de stérilisation ou de désinfection selon au moins une des revendications 1 à 10, **caractérisé** en ce que les dispositifs de canalisation de fluide (6) ont des propriétés de canalisation de fluide qui peuvent varier, et peuvent notamment être fermés.

12. Appareil de stérilisation ou de désinfection selon au moins une des revendications 1 à 11, **caractérisé** en ce que les dispositifs de canalisation de fluide (6) prévus dans la porte (3) communiquent avec d'autres dispositifs de canalisation de fluide disposés à l'intérieur de la chambre, par exemple avec un canal s'étendant dans une zone d'angle inférieure de la chambre et menant à une extrémité de la chambre tournée à l'opposé du dispositif de fermeture.
